# EUROPÄISCHE PATENTSCHRIFT

(11) **EP 4 003 196 B1**
(45) Veröffentlichungstag und Bekanntmachung des Hinweises auf die Patenterteilung: **28.08.2024**
(21) Anmeldenummer: 20753902.4
(22) Anmeldetag: 30.07.2020
(51) Int. Cl.: A61B 17/70

(54) **WIRBELSÄULENIMPLANTATSVERBINDUNGSEINRICHTUNG**
SPINAL COLUMN IMPLANT CONNECTION DEVICE
DISPOSITIF DE RACCORDEMENT D'IMPLANT DE COLONNE VERTÉBRALE

(30) Priorität: 30.07.2019 DE 102019005374
(43) Veröffentlichungstag der Anmeldung: 01.06.2022
(73) Patentinhaber: Signus Medizintechnik GmbH, 63755 Alzenau (DE)
(72) Erfinder: VAN DER POL, Bas, 63755 Alzenau (DE)
(74) Vertreter: Leinweber & Zimmermann
(86) Internationale Anmeldenummer: PCT/EP2020/071578
(87) Internationale Veröffentlichungsnummer: WO 2021/019046

(56) Entgegenhaltungen:
- DE-A1- 102011 113 235
- US-A1- 2009 177 232
- US-A1- 2010 217 334
- US-A1- 2012 253 397

## Beschreibung

Die Erfindung betrifft eine Verbindungseinrichtung zur Verbindung eines Endbereichs einer bereits implantierten ersten Wirbelsäulenstütze, bei welcher eine Mehrzahl von Pedikelschrauben an ihrer Kopfseite über jeweils eine Kopplungseinrichtung mit einer ersten Verbindungsstange gekoppelt sind, mit einem bereits implantierten oder noch zu implantierenden, eine Pedikelschraube aufweisenden und die erste Wirbelsäulenstütze verlängernden Wirbelsäulenstützabschnitt, mit einer ersten Befestigungsanordnung zu ihrer Befestigung an der ersten Verbindungsstange und einer Ankopplung für den verlängernden Stützabschnitt, insbesondere in Form einer mit der Pedikelschraube des verlängernden Stützabschnitts zu koppelnden zweiten Stange oder einer zweiten Befestigungsanordnung zur Befestigung der Verbindungseinrichtung an einer solchen zweiten Stange.

Derartige Verbindungseinrichtungen sind bekannt und kommen bevorzugt dann zum Einsatz, wenn eine bereits bestehende Wirbelsäulenstütze für deren Träger nicht mehr ausreicht, sondern zu verlängern ist, um sich über noch zusätzliche Wirbel zu erstrecken. Ein kompletter Austausch der bestehenden Wirbelsäulenstütze wäre mit einer höheren operativen Belastung verbunden, da die bestehende Verbindungsstange für ihren Ersatz durch eine längere Stange über ihre gesamte Länge freigelegt und aus ihrer eingespannten Lage zu lösen wäre, was gerade bei häufig verwendeten Wirbelsäulenstützen, bei denen durch Lösen der Festlegung der Verbindungsstange auch die u. U. in besonderen Drehlagen eingekoppelten Kopplungszustände der Pedikelschrauben und der Kopplungseinrichtungen aufgehoben würden, erheblichen Wiederherstellungsaufwand mit sich brächte.

Daher wird an eine Verlängerung des bislang implantierten Systems gedacht. Ein hierzu bekanntes System zur Längskopplung zweier Verbindungsstangen ist in US 2008/234743 A1 offenbart, eignet sich zu den o. g. Zwecken jedoch nur bedingt, da es ein Gewinde am kopplungsseitigen Ende beider Stangen voraussetzt, das jedenfalls für die bereits implantierte Stange im Regelfall nicht gegeben sein muss. Ohne solches Gewinde kommt die in WO 2010/120989 A1 vorgestellt Lehre aus. In einer ersten Variante der Lehre dieses Dokuments wird die bestehende Verbindungsstange zwischen den beiden verbindungsseitig nächsten Pedikelschrauben abgeschnitten, und es wird (nur) die äußerste Pedikelschraube entfernt. In diesen Wirbel wird dann eine Pedikelschraube eingeschraubt, deren Kopplungseinrichtung eine seitliche Verlängerung mit einer Rinne hat, in die die Verbindungsstange der bereits implantierten Wirbelsäulenstütze eingelegt und mit einer Schraube befestigt wird. Soll gemäß der Lehre der WO 2010/120989 A1 ein solches Abschneiden der bereits implantierten Verbindungsstange nicht erfolgen, kann eine Verlängerungsstütze, wie in Fig. 14A dieses Dokuments gezeigt, angekoppelt werden, welche in Fig. 6 dieser Anmeldung wiedergegeben ist. Parallel zur bereits bestehenden Verbindungsstange wird eine Bypass-Stange 247 gelegt, die über jeweils eine Verbindungsmanschette mit der bereits implantierten Verbindungsstange und der Verbindungsstange der Verlängerung verbunden wird.

US 2009/0177232 A1 offenbart eine Verbindungseinrichtung gemäß dem Oberbegriff von Anspruch 1. DE 10 2011 113 235 A1 schlägt ein System zur Verlängerung eines Implantats vor, bei dem das bestehende Implantat nicht mehr entfernt werden muss. US 2010/0217334 A1 schlägt ein einfaches System vor, bei dem das Erfordernis, auf jede Pedikelschraube vor Implantierung eine Verbindungsvorrichtung zu platzieren, eliminiert ist.

All diese Systeme haben ihre Vor- und Nachteile. Der Erfindung liegt die Aufgabe zugrunde, eine Verbindungseinrichtung der eingangs genannten Art zu schaffen, die hinsichtlich einer guten Kombination aus baulicher Einfachheit, Einfachheit in ihrer Anbringung/ihrem Einsatz und geringen Belastungen für den Träger verbessert ist.

Diese Aufgabe wird von der Erfindung durch eine Verbindungseinrichtung mit allen Merkmalen von Anspruch 1 gelöst. Insoweit ist einerseits vorgesehen

eine sich an die Kopplungseinrichtung der verbindungsseitig nächstgelegenen Pedikelschraube der bereits implantierten ersten Wirbelsäulenstütze lateral anschmiegende starre Überbrückung eines zwischen ihrer ersten Befestigungsanordnung und der Ankopplung, insbesondere zwischen ihrer ersten und zweiten Befestigungsanordnung bzw. ihrer ersten Befestigungsanordnung und der zweiten Stange liegenden Zwischenbereichs. Die erfindungsgemäße Verbindungseinrichtung erlaubt einen Einsatz ohne Modifizierung der bestehenden implantierten Wirbelsäulenstütze mit einfacher Handhabung bei dennoch zufriedenstellender Qualität der erforderlichen starren Kopplung. Durch das Anschmiegen an die Kopplungseinrichtung haben an der Verbindungseinrichtung angreifende Kräfte nur einen geringen Hebelarm, abgesehen von einem ermöglichten geringeren Raumbedarf der Verbindungseinrichtung.

In einer besonders bevorzugten Ausgestaltung ist das Anschmiegen ein lateral beidseitiges Anschmiegen. Auf diese Weise kann bei immer noch zufriedenstellend geringem Materialaufwand eine erhöhte Stabilität der Verbindungseinrichtung erreicht werden. Ist die Erstreckungsrichtung der zu verlängernden Wirbelsäulenstütze eine Längsrichtung, eine Erstreckungsrichtung der Kopplungseinrichtung eine Höhenrichtung und die dritte Raumrichtung eine Querrichtung, welche zusammen mit der Längsrichtung eine Ebene bildet, in deren Projektion das Anschmiegen der Überbrückung erkennbar ist, so beträgt bevorzugt eine maximale Abmessung der Verbindungseinrichtung in der Querrichtung weniger als 3 cm, bevorzugt weniger als 2,7 cm, weiter bevorzugt weniger als 2,4 cm, und insbesondere weniger als 2 cm.

Des Weiteren ist bevorzugt vorgesehen, dass eine Höhenausdehnung der Überbrückung die Höhe der Kopplungseinrichtung um nicht mehr als 100 %, bevorzugt nicht mehr als 60 %, insbesondere nicht mehr als 30 % von deren Höhenabmessung übersteigt. In einer weiter bevorzugten Ausgestaltung ist das Höhenniveau des oberen Endes der Kopplungseinrichtung nicht höher als das Höhenniveau des oberen Endes der Überbrückung. Im letzteren Fall bedarf es für den Träger jedenfalls entlang der Höhenrichtung keines über den bereits bestehenden Raumbedarf hinausgehenden Raumbedarfs.

In einer besonders bevorzugten Ausgestaltung weist die Verbindungseinrichtung eine dritte Befestigungsanordnung zu ihrer Befestigung an der ersten Verbindungsstange an deren verbindungsseitig freiem Ende auf. Dies erhöht weiter die Stabilität der Verbindung, da in Längsrichtung gesehen eine Befestigung der Verbindungseinrichtung beidseits der Kopplungseinrichtung erfolgt und somit selbst bei unbeabsichtigtem Spiel aufgrund einer unbeabsichtigten Lockerung einer der Befestigungsanordnungen weiterhin eine vergleichsweise stabile Befestigung erhalten bleibt.

Zur Vermeidung Letzteres können die für die Befestigungsanordnungen heranzuziehenden Spannschrauben selbsthemmend gestaltet sein.

Erfindungsgemäß ist die erste Befestigungsanordnung zur Positionierung der Verbindungseinrichtung gegenüber der ersten Wirbelsäulenstütze radial und insbesondere von oben auf den die Kopplungseinrichtung der verbindungsseitig nächstgelegenen Pedikelschraube umfassenden Endbereich aufsetzbar. So kann ein Positionieren im Einsatz durch einen einzigen Aufsetzschritt erfolgen. Die sich an die Kopplungseinrichtung anschließenden Bereiche können dabei eine Führung beim Aufsetzen bilden.

Erfindungsgemäß ist die Aufsetzbarkeit eine Aufklipsbarkeit der ersten und ggf. dritten Befestigungsanordnung mittels einer (jeweiligen) federnden Einrichtung. Dies vereinfacht erneut die Anbringung im Einsatz, da durch das Aufklipsen werkzeugfrei eine bereits richtig positionierte, jedoch nicht mehr unbeabsichtigt von der Verbindungsstange lösbare Positionierung bereitgestellt wird. Durch ein insbesondere nahezu komplementärförmiges Anschmiegen wird zudem auch bereits die richtige Axialpositionierung eingenommen. Weiter erfindungsgemäß ist die federnde Einrichtung derart gestaltet, dass eine seitliche Begrenzung eines die erste Verbindungsstange aufnehmenden Aufnahmebereichs, in Form einer Aufnahmerinne, durch die Innenfläche eines federnden Arms gebildet ist, und die andere seitliche Begrenzung an der (in lateraler Richtung/Querrichtung) gegenüberliegenden Seite durch eine die Funktion eines feststehenden Arms übernehmende Seitenstruktur der ersten Befestigungsanordnung.

Dieser Gesichtspunkt der einfachen Herstellung einer Positionierung wird von der Erfindung auch eigenständig und unabhängig von der Art der Gestaltung der starren Überbrückung hinsichtlich eines Anschmiegens als eigenständig einsetzbar und schutzwürdig offenbart.

Die Erfindung betrifft somit ebenfalls eine Verbindungseinrichtung zur Verbindung eines Endbereichs einer bereits implantierten ersten Wirbelsäulenstütze, bei welcher eine Mehrzahl von Pedikelschrauben an ihrer Kopfseite über jeweils eine Kopplungseinrichtung mit einer ersten Verbindungsstange gekoppelt sind, mit einem bereits implantierten oder noch zu implantierenden, eine Pedikelschraube aufweisenden und die erste Wirbelsäulenstütze verlängernden Wirbelsäulenstützabschnitt, mit einer ersten Befestigungsanordnung zu ihrer Befestigung an der ersten Verbindungsstange und einer Ankopplung für den verlängernden Stützabschnitt, insbesondere in Form einer mit der Pedikelschraube des verlängernden Stützabschnitts zu koppelnden zweiten Stange oder einer zweiten Befestigungsanordnung zur Befestigung der Verbindungseinrichtung an einer solchen zweiten Stange, bei der die erste Befestigungsanordnung zur Positionierung der Verbindungseinrichtung gegenüber der ersten Wirbelsäulenstütze radial und insbesondere von oben auf den die Kopplungseinrichtung der verbindungsseitig nächstgelegenen Pedikelschraube umfassenden Endbereich mittels einer federnden Einrichtung der ersten Befestigungsanordnung und/oder einer auf der anderen Seite der Kopplungseinrichtung zu befestigenden dritten Befestigungsanordnung bewirkten Aufklipsbarkeit der ersten und/oder dritten Befestigungsanordnung aufsetzbar ist.

Es versteht sich, dass die voranstehenden und/oder nachfolgenden weiteren Merkmale der Verbindungseinrichtung auch für diese unabhängige Variante der aufklipsbaren Verbindungseinrichtung gemeinsam zum Einsatz kommen können. Ein Festspannen der ersten Verbindungsstange an der ersten und/oder dritten Befestigungsanordnung erfolgt mit Spannmitteln, die Teil der Verbindungseinrichtung sind.

In diesem Zusammenhang ist vorgesehen, dass eine Spannschraube vorgesehen ist, die bezüglich eines die erste Verbindungsstange aufnehmenden Aufnahmebereichs der ersten und/oder dritten Befestigungsanordnung außermittig angeordnet ist. Die Spannschraube ist in eine gewindebehaftete Bohrung einschraubbar, welche nicht zu dem in Form einer Aufnahmerinne gebildeten Aufnahmebereich für die erste Verbindungsstange hindurch reicht. Eine axiale Krafteinleitungsrichtung beim Einschrauben der Spannschraube wird somit nicht mehr zentral bezüglich der im Querschnitt gesehen Mitte der Verbindungsstange 60 aufgenommen und wirkt der Federwirkung der federnden Einrichtung entgegen bis hin zu deren Aufheben und schließlich Festspannen der Verbindungsstange in dem diese aufnehmenden Bereich der Befestigungsanordnung. Für Letzteres klemmen bevorzugt ein federnder Arm und eine die Funktion eines feststehenden Arms übernehmende Seitenstruktur der ersten Befestigungsanordnung die erste Verbindungsstange in der orthogonal zur axialen Krafteinleitungsrichtung verlaufenden lateralen Richtung ein.

Sofern die zweite Stange für die Verlängerung der Wirbelsäulenstütze nicht bereits Bestandteil der Verbindungseinrichtung ist, kann in einer bevorzugten Ausführungsform die zweite Befestigungsanordnung zum in deren Längsrichtung axialen Einschieben der zweiten Stange ausgelegt sein und insbesondere in Form einer axialen Bohrung in einem Materialblock gebildet sein. Dies erlaubt ein axiales Einschieben der zweiten Spange beispielsweise bei bereits aufgeklipster Verbindungseinrichtung. In der axialen Bohrung kann die zweite Stange dann durch Feststellschrauben fixiert werden. Es versteht sich, dass auch laterale Einschubmöglichkeiten denkbar sind, wie U-förmige Rinnen. Bevorzugt fallen die Erstreckungsrichtungen der Stangen zusammen, es sind jedoch auch gewinkelte Anordnungen denkbar, insbesondere auch komplexere Kopplungen, die eine polyaxiale Winkeleinstellung erlauben.

In einer bevorzugten Ausführungsform ist die maximale Querabmessung der Verbindungseinrichtung geringer als 1/3, bevorzugt geringer als 2/7 ihrer axialen Abmessung (gerechnet ohne ggf. realisierte zweite Stange), und/oder geringer als 440 %, insbesondere 360 % des Durchmessers des Aufnahmebereichs für die erste Verbindungsstange. Es kann bei ausreichender Qualität der Starrheit der Überbrückung eine kompakte Konstruktion bereitgestellt werden. Durch das Anschmiegen der Überbrückung kann in einer bevorzugten Ausgestaltung des lateral beidseitigen Anschmiegens eine manschettenartige oder bandagenartige Verwendung geschaffen werden, mit an der Längsstelle maximaler Querabmessung geringerer Querabmessung des umschmiegenden Anteils gegenüber dem umschmiegten Anteil. Bei bevorzugter Realisierung des Umschmiegens durch ein hülsenartiges Teil wird es bevorzugt, dass eine Wandstärke der Hülsenwand im Bereich der maximalen Querabmessung der Verbindungseinrichtung geringer ist als 40 %, bevorzugt geringer als 30 %, insbesondere geringer als 20 % der Querabmessung des von der Hülse umschlossenen Bereichs. Bevorzugt ist das hülsenartige Teil einstückig mit der ersten Befestigungsanordnung verbunden.

Von der Erfindung ebenfalls bereitgestellt wird ein Verlängerungsset zur Verlängerung einer bereits implantierten ersten Wirbelsäulenstütze, mit einer Verbindungseinrichtung nach einem der vorgenannten Aspekte und mit wenigstens einer Pedikelschraube, einer zweiten Stange (sofern diese nicht ggf. bereits Teil der Verbindungseinrichtung ist) und wenigstens einer Kopplungseinrichtung zur Bildung einer starren Verbindung zwischen der Pedikelschraube und der zweiten Stange.

Ebenfalls wird eine Wirbelsäulenstütze als Ganzes, aufweisend eine erste Wirbelsäulenstütze mit einer Mehrzahl von Pedikelschrauben, die an ihrer Kopfseite über jeweils eine Kopplungseinrichtung mit einer ersten Verbindungsstange gekoppelt sind, und eine mittels einer Verbindungseinrichtung nach einem der vorgenannten Aspekte verbundene Verlängerung, unter Schutz gestellt.

Die Erfindung stellt auch das Herrichten einer solchen Verbindungseinrichtung für ihren bevorstehenden Einsatz der Schaffung einer Verlängerung einer ersten Wirbelsäulenstütze unter Schutz. Diese kann mehrere Aspekte beinhalten, zum einen die (ohnehin vorgesehene) Ausgestaltung der Verbindungseinrichtungen materialtechnisch aus biokompatiblen Materialien (wie etwa Titan, rostfreier Stahl oder auch Kunststoffe, etwa biokompatible Polymere, die dem Fachmann auf dem Gebiet der Implantologie bekannt sind), wie auch die sinnfällige Herrichtung durch Desinfektionsschritte wie z. B. Autoklavieren, die passende Zusammenstellung passend zu Stützstangen der bereits implantierten ersten Wirbelsäulenstütze und/oder die Bereitlegung der zusammengestellten Teile für den das Implantieren vornehmenden Chirurgen. Hinsichtlich der Pedikelschrauben und ihren Kopplungseinrichtungen an Stangen, wie sie dem Fachmann bekannt sind und in dieser Anmeldung mehrfach angesprochen sind, wird beispielsweise auf Techniken wie in der WO 2009/015100 A2 oder EP 2 581 057 B1 verwiesen.

Des Weiteren ist auch vorgesehen, ein Sortiment, aufweisend wenigstens zwei, insbesondere wenigstens vier Verbindungseinrichtungen, nach einem der vorgenannten Aspekte bereitzustellen, wobei die ersten Befestigungsanordnungen von wenigstens zwei Verbindungseinrichtungen voneinander unterschiedlich ausgeführt sind, um an Verbindungsstangen unterschiedlicher Querabmessung befestigt zu werden, auf Kopplungseinrichtungen unterschiedlicher Größendimension aufsetzbar zu sein, und/oder wenigstens zwei Verbindungseinrichtungen zweite Stangen unterschiedlicher Länge aufweisen. Mit einem derartigen Sortiment kann flexibel auf Fälle reagiert werden, in denen nicht von einer bereits implantierten Wirbelsäulenstütze bekannter und einheitlicher Abmessung ihrer Teile ausgegangen werden kann, wodurch sich die Flexibilität des Einsatzes der Verbindungseinrichtungen nochmals erhöht.

Weitere Merkmale, Einzelheiten und Vorteile der Erfindung ergeben sich aus der nachfolgenden Beschreibung mit Bezug auf die beigefügten Figuren, von denen
- Fig. 1a: eine Perspektivansicht einer Verbindungseinrichtung einer ersten Ausführungsform ist,
- Fig. 1b: eine Draufsicht auf die Verbindungseinrichtung von Fig. 1a ist,
- Fig. 2a: eine entlang der Linie A-A aus Fig. 1a genommene Schnittansicht umfasst,
- Fig. 2b: eine entlang der Linie B-B in Fig. 1b genommene Schnittansicht umfasst,
- Fig. 3a: die Verbindungseinrichtung aus Fig. 1a in ihrem verbindenden Einsatz in einer Draufsicht zeigt,
- Fig. 3b: eine Schnittansicht entlang der Linie A-A aus Fig. 3a umfasst,
- Fig. 3c: eine Schnittansicht entlang der Linie B-B in Fig. 3a umfasst,
- Fig. 4: eine Verbindungseinrichtung einer zweiten Ausführungsform zeigt,
- die Fig. 5a, 5b, 5c: den Fig. 3a, 3b und 3c für diese zweite Ausführungsform entsprechen, und

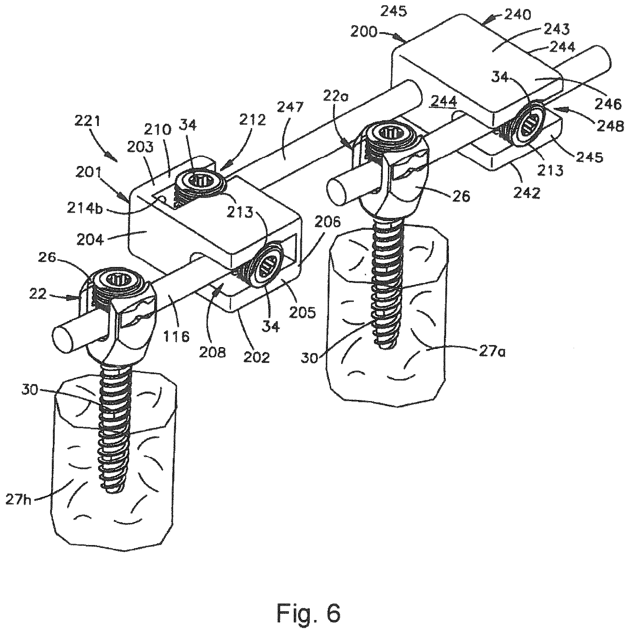

- Fig. 6: eine aus dem Stand der Technik bekannte Verbindungseinrichtung zeigt.

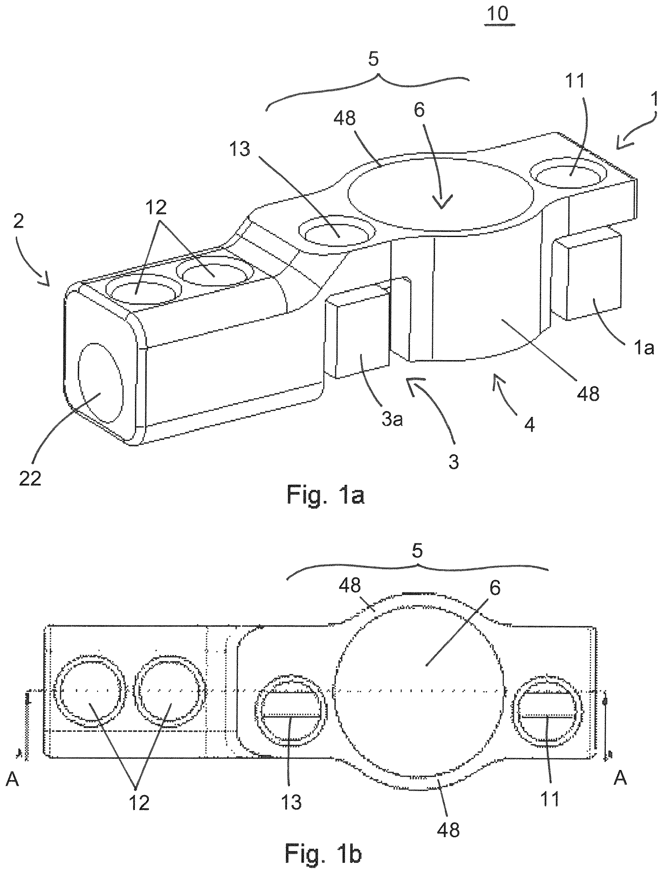

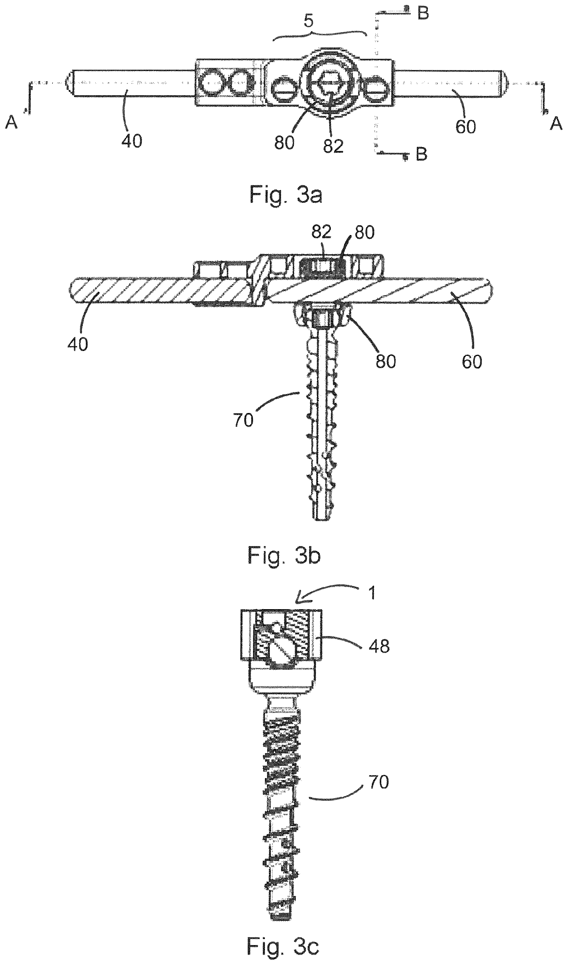

Eine Verbindungseinrichtung 10 einer ersten Ausführungsform ist in Fig. 1a in perspektivischer Ansicht dargestellt. In Fig. 1a ganz rechts erkennt man eine erste Befestigungsanordnung 1 mit einem Federarm 1a und einer mit nicht dargestelltem Gewinde versehenen Bohrung 11 zum Einschrauben einer nicht dargestellten Spannschraube. Die erste Befestigungsanordnung 1 dient der Befestigung der Verbindungseinrichtung 10 an einer Verbindungsstange 60 (Fig. 3a) einer Wirbelsäulenstütze.

Im in Fig. 1a linken Endbereich weist die Verbindungseinrichtung 10 eine zweite Befestigungsanordnung 2 auf. Diese hat eine in das Material des Endbereichs eingelassene Bohrung 22, die sich in Längsrichtung der Verbindungseinrichtung 10 erstreckt, und in Höhenrichtung verlaufende gewindebehaftete Durchgangsbohrungen 12, die bis zur Bohrung 22 reichen. Auf diese Weise kann durch nicht dargestellte Feststellschrauben, die in die Gewindebohrungen 12 eingeschraubt werden, eine in die Bohrung 22 eingesteckte Stange fixiert werden. Die zweite Befestigungsanordnung 2 dient somit der Befestigung einer Stange 40, die Teil einer die die Verbindungsstange 60 aufweisende Wirbelsäulenstütze verlängernde Stütze bildet, obgleich in Fig. 3b nur die Stange 40 selbst dargestellt ist. Ein Zwischenbereich 5 zwischen der ersten Befestigungsanordnung 1 und der zweiten Befestigungsanordnung 2 weist benachbart der ersten Befestigungsanordnung 1 einen hülsenartigen Bereich 4 auf, dessen Innenraum 6 in diesem Ausführungsbeispiel zylindrisch gestaltet ist, wobei sich die Zylinderachse in Höhenrichtung erstreckt. Wie aus Fig. 1a erkennbar ist, ist der hülsenartige Bereich 4 einstückig mit der ersten Befestigungsanordnung 1 verbunden. Im Verbindungseinsatz wird im Innenraum 6 eine Kopplungseinrichtung 80 aufgenommen, die eine Pedikelschraube 70 der Wirbelsäulenstütze mit deren Verbindungsstange 60 fixierend koppelt. Wie aus Fig. 1a erkennbar ist, sind die erste Befestigungsanordnung 1 und die zweite Befestigungsanordnung 2 einstückig über den Zwischenbereich 5 miteinander verbunden.

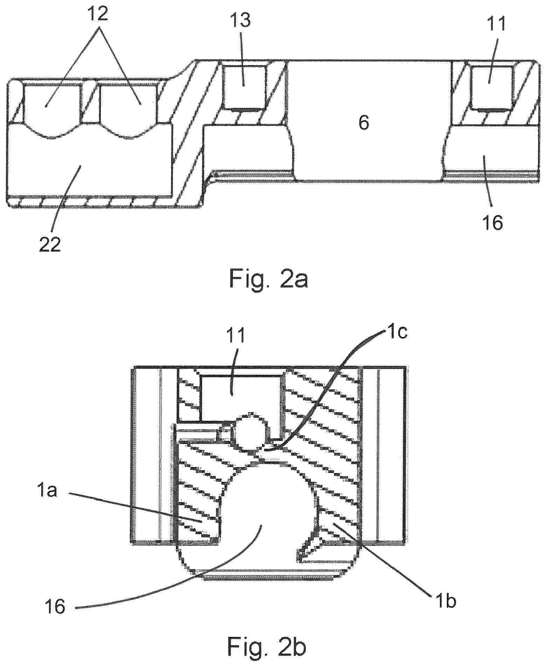

Zwischen dem hülsenartigen Bereich 4 und der zweiten Befestigungsanordnung 2 ist noch eine dritte Befestigungsanordnung 3 mit Federarm 3a und gewindebehafteter Bohrung 13 angeordnet, welche in diesem Ausführungsbeispiel mit der ersten Befestigungsanordnung 1 baugleich ist und nachstehend mit Bezug auf die Fig. 2 erläutert wird. Die dritte Befestigungsanordnung 3 dient wie die Befestigungsanordnung 1 der Festlegung der Verbindungseinrichtung an der Verbindungsstange 60 einer bereits implantierten Wirbelsäulenstütze, allerdings auf der der Befestigungsanordnung 1 abgewandten Seite der Kopplungseinrichtung 80, am freien Ende der Verbindungsstange 60. Die erste und dritte Befestigungsanordnung 1, 3 umgeben somit die Kopplungseinrichtung der zu verlängernden Wirbelsäulenstütze sandwichartig, und der hülsenartige Bereich 4 bzw. dessen laterale Wände 48 schmiegen sich dabei an die Kopplungseinrichtung 80 an. Wie aus Fig. 1a zu erkennen ist, sind die erste Befestigungsanordnung 1 und die dritte Befestigungsanordnung 3 einstückig über den hülsenartigen Bereich 4 miteinander verbunden.

In Fig. 2a ist zu erkennen, dass eine Aufnahmerinne 16 für die Verbindungsstange 60 in diesem Ausführungsbeispiel axial zentral und nahezu koaxial zur Bohrung 22 verläuft. Im Bereich der ersten und dritten Befestigungsanordnung ist die Mündungsöffnung der Rinne 16 in unbelastetem Zustand der Federarme 1a, 3a geringfügig enger als der Durchmesser der Rinne 16. Die Befestigungsanordnungen 1, 3 können somit unter elastischem Nachgeben ihrer federnden Arme 1a, 3a auf die Verbindungsstange 60 aufgeklipst werden, während bei dieser Aufklipsbewegung der hülsenartige Bereich 4 über die Kopplungseinrichtung 80 gestülpt wird.

Die gewindebehafteten Bohrungen 11 und 13 reichen nicht bis zur Rinne 16 hindurch (Fig. 2). Zudem sind sie, wie in Fig. 1b und Fig. 2b zu erkennen ist, bezüglich der Mittelachse der Verbindungseinrichtung 10 außermittig in Richtung auf die Federarme 1a, 3a hin verlagert angeordnet, so dass beim Einschrauben der Spannschrauben zunächst die federnde Wirkung der Federarme 1a, 3a aufgehoben wird und in diesem Zustand ein Wiederabnehmen der Verbindungseinrichtung 10 von der Wirbelsäulenstütze (Stange 60) gesperrt ist. Bei weiterem Anziehen bzw. Festziehen der Spannschrauben wird die Verbindungseinrichtung 10 starr und fest mit der Verbindungsstange 60 der bereits implantierten Wirbelsäulenstütze verbunden. Über die zweite Befestigungsanordnung 2 ist somit auch die Stange 40 des Verlängerungsabschnitts dieser Wirbelsäulenstütze starr und fest mit der zu verlängernden Wirbelsäulenstütze verbunden. Wie aus Fig. 2b erkennbar ist, sind das Gewinde für Bohrung 11 und die einander gegenüberliegenden seitlichen Begrenzungen der Rinne 16 einstückig miteinander verbunden.

In diesem Ausführungsbeispiel ist die Funktion der Aufklipsbarkeit der Befestigungsanordnung 1 realisiert, indem, im Querschnitt zur Rinne 16 gesehen, deren Begrenzung an einer Seite durch die Innenfläche des federnden Arms 1a gebildet ist, an der gegenüberliegenden Seite durch eine die Funktion des feststehenden Arms 1b übernehmende Seitenstruktur der Befestigungsanordnung 1, und die beiden Arme 1a und 1b nur durch eine Materialbrücke 1c geringer Dicke miteinander verbunden sind. Material und Dimensionierung der Materialbrücke 1c sind jedoch so aufeinander abgestimmt, dass ein Abbrechen des federnden Arms 1a bei zufälliger unbeabsichtigter Krafteinwirkung vermieden wird. Wie aus Fig. 2b erkennbar ist, liegen sich die seitlichen Begrenzungen 1a, 1b der Rinne bezüglich einer Querrichtung bzw. lateraler Richtung gegenüber, die orthogonal zur gewindebehafteten Bohrung 11 verläuft. Wie oben bereits erläutert, ist die gewindebehaftete Bohrung 11 nicht mittig bezüglich der seitlichen Begrenzungen 1a, 1b angeordnet, sondern außermittig in Querrichtung auf den Federarm 1a hin verlagert.

In den Figurendarstellungen der Fig. 3 sind neben den dargestellten Schnittdarstellungen auch noch in Projektion erkennbare Bestandteile mit abgebildet, etwa die Pedikelschraube 70 in Fig. 3c oder die Seitenbereiche 48 des hülsenartigen Bereichs 4, um die Lage zu diesen Teilen/Gegenständen erkennen zu können. So ist insbesondere aus Fig. 3a gut erkennbar, dass sich die an die Verbindungsstange 60 ankoppelnde Seite der Verbindungseinrichtung 10 manschetten- oder bandagenartig um den nicht nur die Verbindungsstange 60, sondern auch die Kopplungseinrichtung 80 aufweisenden Endbereich der bereits implantierten Wirbelsäulenstütze legt. So beträgt in diesem Ausführungsbeispiel die maximale Querabmessung der Verbindungseinrichtung 10, gebildet hier durch den transversalen Abstand der beiden Seitenwände 48 des hülsenförmigen Bereichs 4, nur etwa das 2,5fache des Durchmessers der Verbindungsstange 60 bzw. der zu deren Aufnahme ausgelegten Rinne 16.

Die Verbindungseinrichtung 10 kann auf diesen Endbereich einfach aufgeknipst werden, ohne dass es einer Manipulationen des Endbereichs an Stange 60 und/oder Kopplungseinrichtung 80 bedarf, auch keines Lösens der Feststellschraube 82 der Kopplungseinrichtung 80. Letztere kann entsprechend den in den Jahren verwendeten Techniken gestaltet sein, die beispielsweise in WO 2009/015100 A2 oder EP 2 581 057 B1 beschrieben sind, mit beispielsweise einer Tulpe, einem in der Tulpe aufgenommenen Sattel, in dem der Kopf der Pedikelschraube 70 aufgenommen ist, und der über die Verbindungsstange 60 Spanndruck auf den Sattel ausübenden Schraube 82 zur festen Kopplung der als insbesondere Polyaxialschraube ausgelegten Schraube 70 in gewünschter Winkellage zur Verbindungsstange 60. Gleichartige Systeme können dann auch für den verlängernden Wirbelsäulenstützenabschnitt mit der Stange 40 zum Einsatz kommen.

In Höhenrichtung gesehen kann, wie in Fig. 3b gezeigt ist, der obere Abschnitt der Kopplungseinrichtung 80 gänzlich im Innenraum 6 des hülsenartigen Bereichs 4 aufgenommen sein. Auf diese Weise kann eine vergleichsweise ebene Oberflächenseite der Verbindungseinrichtung im Einsatzzustand gebildet sein.

Es ist für den Fachmann erkennbar, dass zu dieser Ausführungsform diverse alternative konstruktive Gestaltungen denkbar sind, so müssten die Achsen für die Stangen 40, 60 nicht kollinear, noch nicht einmal parallel sein, falls gewinkelte Verbindungen erforderlich werden. Die zweite Befestigungsanordnung 2 könnte auch anders gestaltet sein, etwa ähnlich der ersten Befestigungsanordnung oder mit einer seitlichen Einführrinne anstelle der Sacklochbohrung 22. Das manschettenartige Anschmiegen könnte auch durch ein seitliches Aufklipsen auf die Verbindungsstange 60 erreicht werden, hierzu wäre der hülsenartige Bereich 4 passend zu einer seitlichen Anbringung zu modifizieren. Bei entsprechend steifer Ausgestaltung kann auch einer der Seitenarme 48 des hülsenartigen Bereichs 4 ausreichen. Die nach unten ragenden flanschartigen Endbereiche der Seitenwände 48 könnten z. B. kürzer ausfallen, Einschnitte aufweisen, oder die Struktur des hülsenartigen Bereichs durch eine deren oberem Bereich entsprechende Ringstruktur ersetzt werden. Im Falle sehr kurz überstehender freier Enden der Verbindungsstange 60 wird auch an Varianten gedacht, an denen die dritte Befestigungsanordnung auf die Seite der ersten Befestigungsanordnung verlagert wird oder die erste Befestigungsanordnung alleine für eine ausreichende Ankopplung an die Verbindungsstange 60 sorgt.

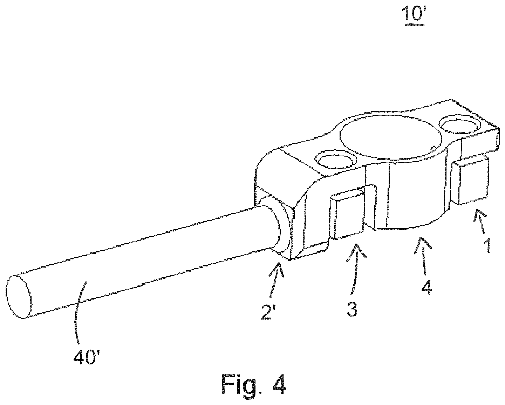

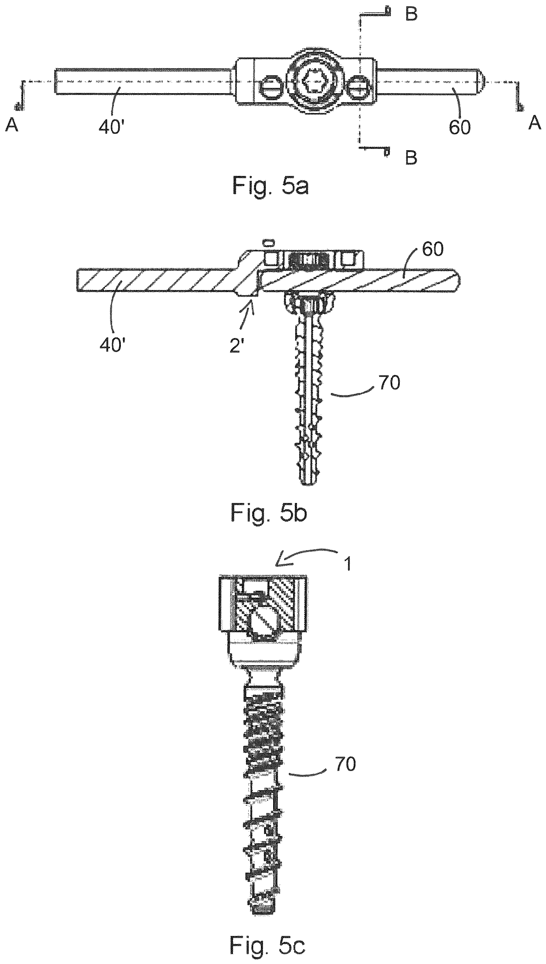

Auch kann die Stange des Verlängerungsabschnitts bereits ein fest angekoppelter Bestandteil der Verbindungseinrichtung sein. Eine derartige Ausführungsform ist in Fig. 4 in schematischer Darstellung gezeigt. Man erkennt, dass bei der dort gezeigten Verbindungseinrichtung 10' die zur Verlängerung der Stange 60 wirkende Stange 40' bereits einstückig angebunden ist, etwa über einen der dritten Befestigungsanordnung 3 angrenzenden Flansch 2' der Verbindungseinrichtung 10'. Wie aus Fig. 4 erkennbar ist, sind der Flansch 2' und die erste Befestigungsanordnung 1 über den Zwischenbereich 5 einstückig miteinander verbunden. Auf diese Weise können auch mehrere Verbindungseinrichtungen 10' mit Stangen 40' unterschiedlicher Länge bereitgestellt werden, je nach Bedarf an Längenabmessung der Verlängerung. Die übrige Gestaltung der Verbindungseinrichtung 10' kann derjenigen der Verbindungseinrichtung 10 aus Fig. 1a entsprechen, insoweit werden auch gleichartige Bezugszeichen zur Bezeichnung gleichartiger Teile verwendet, und die Darstellungen in den Fig. 5a, 5b und 5c entsprechen, in diesem Ausführungsbeispiel funktional wie konstruktiv, der Darstellung der Figurengruppe 3 der ersten Ausführungsform, eben bis auf die bereits vorgesehene Stange 40'. Es versteht sich, dass auch Mischformen einsetzbar sind, beispielsweise könnte die Verbindung zwischen Stange 40' und Flansch 2' nicht einstückig gebildet sein, sondern eine Stange 40" in einen Flansch 2" einschraubbar sein.

Materialtechnisch sind die Bestandteile der Verbindungseinrichtung aus einem biokompatiblen Material gefertigt, wie etwa rostfreier Stahl, Titan oder biokompatible Polymere, wie sie dem Fachmann als solche bekannt sind.

Ein Herrichten der Verbindungseinrichtung für den Einsatz kann einen Desinfizierschritt und/oder Autoklavierschritt umfassen sowie die passende Bereitstellung nicht nur der Verbindungseinrichtung, sondern auch der gesamten verlängernden Wirbelsäulenstütze mit Verbindungsstange 40 (40', 40") passender Länge und Pedikelschrauben/Kopplungseinrichtungen ähnlich wie der in den Fig. 3 und 5 gezeigten Pedikelschraube 70 und Kopplungseinrichtung 80, zu deren konkreten Gestaltungen die gängigen Konstruktionen offenstehen wie etwa die in den Dokumenten, auf die oben Bezug genommen wurde, Dargestellten.

## Patentansprüche

1. Verbindungseinrichtung (10; 10') zur Verbindung eines Endbereichs einer bereits implantierten ersten Wirbelsäulenstütze, bei welcher eine Mehrzahl von Pedikelschrauben (70) an ihrer Kopfseite über jeweils eine Kopplungseinrichtung (80) mit einer ersten Verbindungsstange (60) gekoppelt sind, mit einem bereits implantierten oder noch zu implantierenden, eine Pedikelschraube aufweisenden und die erste Wirbelsäulenstütze verlängernden Wirbelsäulenstützabschnitt, mit
einer ersten Befestigungsanordnung (1) zu ihrer Befestigung an der ersten Verbindungsstange (60) und
einer Ankopplung für den verlängernden Stützabschnitt, insbesondere in Form einer mit der Pedikelschraube des verlängernden Stützabschnitts zu koppelnden zweiten Stange (40') oder einer zweiten Befestigungsanordnung (2) zur Befestigung der Verbindungseinrichtung (10) an einer solchen zweiten Stange (40), und weiter mit
einer sich an die Kopplungseinrichtung (80) der verbindungsseitig nächstgelegenen Pedikelschraube (70) der bereits implantierten ersten Wirbelsäulenstütze lateral anschmiegenden starren Überbrückung eines zwischen ihrer ersten Befestigungsanordnung und der Ankopplung liegenden Zwischenbereichs (5), wobei die erste Befestigungsanordnung (1) zur Positionierung der Verbindungseinrichtung gegenüber der ersten Wirbelsäulenstütze radial auf den die Kopplungseinrichtung (80) der verbindungsseitig nächstgelegenen Pedikelschraube umfassenden Endbereich mittels einer federnden Einrichtung der ersten Befestigungsanordnung bewirkten Aufklipsbarkeit aufsetzbar ist, und mit Spannmitteln zum Festspannen der ersten Verbindungsstange (60) an der ersten (1) Befestigungsanordnung,
**dadurch gekennzeichnet, dass** die federnde Einrichtung derart gestaltet ist, dass eine seitliche Begrenzung eines die erste Verbindungsstange (60) aufnehmenden, in Form einer Aufnahmerinne (16) gebildeten Aufnahmebereichs durch die Innenfläche eines federnden Arms (1a) gebildet ist, und die andere seitliche Begrenzung an der gegenüberliegenden Seite durch eine die Funktion eines feststehenden Arms übernehmende Seitenstruktur der ersten Befestigungsanordnung,
wobei eine in axialer Richtung eingeleitete Kraft beim Einschrauben einer in eine gewindebehaftete, nicht zu der Aufnahmerinne (16) hindurchreichenden Bohrung (11) einschraubbaren Spannschraube zum Festspannen der Verbindungsstange (60) durch deren außenmittige Anordnung bezüglich der Aufnahmerinne nicht zentral bezüglich der im Querschnitt gesehen Mitte der Verbindungsstange aufgenommen wird.

2. Verbindungseinrichtung nach Anspruch 1, bei der das Anschmiegen ein lateral beidseitiges Anschmiegen ist.

3. Verbindungseinrichtung nach Anspruch 1 oder 2, bei der eine Höhenausdehnung der Überbrückung die Höhe der Kopplungseinrichtung um nicht mehr als 100 %, bevorzugt nicht mehr als 60 %, insbesondere nicht mehr als 30 % von deren Höhenabmessung übersteigt.

4. Verbindungseinrichtung nach einem der vorhergehenden Ansprüche, mit einer dritten Befestigungsanordnung (3) zu ihrer Befestigung an der ersten Verbindungsstange (60) an deren verbindungsseitig freiem Ende.

5. Verbindungseinrichtung nach einem der vorhergehenden Ansprüche, bei der die Aufsetzbarkeit auf den Endbereich von oben gegeben ist.

6. Verbindungseinrichtung nach einem der Ansprüche 4 oder 5, bei dem die Aufsetzbarkeit eine Aufklipsbarkeit auch der dritten (3) Befestigungsanordnung mittels einer federnden Einrichtung (3a) beinhaltet.

7. Verbindungseinrichtung nach einem der vorangehenden Ansprüche, mit einer weiteren Spannschraube, die bezüglich eines die erste Verbindungsstange (60) aufnehmenden Aufnahmebereichs (16) der dritten Befestigungsanordnung außermittig angeordnet ist.

8. Verbindungseinrichtung nach einem der vorhergehenden Ansprüche, bei dem die zweite Befestigungsanordnung (2) zum in deren Längsrichtung axialen Einschieben der zweiten Stange (40) ausgelegt ist und insbesondere in Form einer axialen Bohrung in einem Materialblock gebildet ist.

9. Verbindungseinrichtung nach einem der vorhergehenden Ansprüche, die zu einer im Wesentlichen kollinearen Lage der ersten Verbindungsstange (60) und der zweiten Stange (40; 40') ausgelegt ist.

10. Verbindungseinrichtung nach einem der vorhergehenden Ansprüche, deren maximale Querabmessung geringer ist als 1/3, bevorzugt geringer ist als 2/7 ihrer axialen Abmessung, gerechnet ohne ggf. realisierte zweite Stange (40'), und/oder geringer ist als 440 %, insbesondere 360 % des Durchmessers des Aufnahmebereichs (16) für die erste Verbindungsstange.

11. Verlängerungsset zur Verlängerung einer bereits implantierten ersten Wirbelsäulenstütze, mit einer Verbindungseinrichtung (10; 10') nach einem der vorhergehenden Ansprüche und mit wenigstens einer Pedikelschraube, einer zweiten Stange (40; 40'), diese ggf. bereits als Teil der Verbindungseinrichtung, und wenigstens einer Kopplungseinrichtung zur Bildung einer starren Verbindung zwischen der Pedikelschraube und der zweiten Stange (40; 40').

12. Wirbelsäulenstütze, aufweisend eine erste Wirbelsäulenstütze mit einer Mehrzahl von Pedikelschrauben, die an ihrer Kopfseite über jeweils eine Kopplungseinrichtung (80) mit einer ersten Verbindungsstange (60) gekoppelt sind, **gekennzeichnet durch** eine mittels einer Verbindungseinrichtung (10; 10') nach einem der Ansprüche 1 bis 10 verbundene Verlängerung.

13. Herrichten eines Verlängerungssets nach Anspruch 11, aus einem biokompatiblen Material, für ihren bevorstehenden Einsatz der Schaffung einer Verlängerung einer bereits implantierten Wirbelsäulenstütze.

14. Sortiment, aufweisend wenigstens zwei, insbesondere wenigstens vier Verbindungseinrichtungen nach einem der Ansprüche 1 bis 10, wobei die erste Befestigungsanordnungen von wenigstens zwei Verbindungseinrichtungen voneinander unterschiedlich ausgeführt sind, um an Verbindungsstangen unterschiedlicher Querabmessung befestigt zu werden, auf Kopplungseinrichtungen unterschiedlicher Größendimension aufsetzbar zu sein, und/oder wenigstens zwei Verbindungseinrichtungen zweite Stangen (40') unterschiedlicher Länge aufweisen.

## Claims

1. A connecting device (10; 10') for connecting an end region of an already implanted first spinal column support, in which a plurality of pedicle screws (70) are coupled at their head side to a first connecting rod (60) by means of a coupling device (80) in each case, comprising an already implanted or yet to be implanted spinal column support section which has a pedicle screw and which lengthens the first spinal column support, comprising
a first fastening arrangement (1) for fastening and clamping the connecting device to the first connecting rod (60) and
a coupling for the lengthening support section, in particular in the form of a second rod (40') to be coupled to the pedicle screw of the lengthening support section or in the form of a second fastening arrangement (2) for fastening the connecting device (10) to such a second rod (40), and further comprising
a rigid bridgeover of an intermediate region (5) between the first fastening arrangement of the connecting device and the coupling, said bridgeover laterally nestling against the coupling device (80) of the closest pedicle screw (70) of the already implanted first spinal column support on the connection side, wherein, for the purposes of positioning the connecting device in relation to the first spinal column support, the first fastening arrangement (1) is, by means of a resilient device (1a) of the first fastening arrangement providing clippability, able to be attachable radially on the end region comprising the coupling device (80) of the closest pedicle screw on the connection side, and comprising clamping means for clamping the first connecting rod (60) to the first (1) fastening arrangement, **characterized in that** the resilient device is designed in such a way that one lateral boundary of a receiving region which receives the first connecting rod and is in the form of a receiving channel formed by the inner face of a resilient arm, and the other lateral boundary is formed on the opposite side by a side structure of the first fastening arrangement which adapts the function of a fixed arm,
wherein a force applied in axial direction, when a clamping screw is screwed into a threaded bore which does not extend through to the receiving region, is not centrally with respect to the middle of the first connecting rod (60), as seen in cross section, by off-center arrangement of the clamping screw with respect to the receiving channel.

2. The connecting device as claimed in claim 1, wherein the nestling is a lateral nestling at both lateral sides.

3. The connecting device as claimed in claim 1 or 2, wherein a vertical extent of the bridgeover does not exceed the height of the coupling device by more than 100%, preferably by not more than 60%, in particular by not more than 30% of the vertical dimension of the latter.

4. The connecting device as claimed in any one of the preceding claims, comprising a third fastening arrangement (3) for fastening said connecting device to the first connecting rod (60) at the free end thereof on the connection side.

5. The connecting device as claimed in in any one of the preceding claims, wherein the attachability is provided from above.

6. The connecting device as claimed in claim 4 or 5, wherein also the attachability of the third (3) fastening arrangement is a clippability by means of a resilient device (3a).

7. The connecting device as claimed in any one of the preceding claims, comprising also a clamping screw which is arranged off-centered in relation to a receiving region (16) of the third fastening arrangement which receives the first connecting rod (60).

8. The connecting device as claimed in any one of the preceding claims, wherein the second fastening arrangement (2) is designed for an axial insertion of the second rod (40) in its longitudinal direction and in particular is formed in the form of an axial bore in a block of material.

9. The connecting device as claimed in any one of the preceding claims, designed for substantially collinear relative positioning of the first connecting rod (60) and the second rod (40; 40').

10. The connecting device as claimed in any one of the preceding claims, the maximum transverse dimension of which is less than 1/3, preferably less than 2/7 of its axial dimension, calculated without an optionally realized second rod (40'), and/or less than 440%, in particular 360% of the diameter of the receiving region (16) for the first connecting rod

11. An extension set for lengthening an already implanted first spinal column support, comprising a connecting device (10; 10') as claimed in any one of the preceding claims and comprising at least one pedicle screw, a second rod (40; 40'), the latter optionally already as part of the connecting device, and at least one coupling device for forming a rigid connection between the pedicle screw and the second rod (40; 40').

12. A spinal column support, having a first spinal column support with a plurality of pedicle screws, which are coupled at their head side to a first connecting rod (60) by way of a coupling device (80) in each case, **characterized by** an extension connected by means of a connecting device (10; 10') as claimed in any one of claims 1 to 10.

13. The preparation of an extension set as claimed in claim 11, made of a biocompatible material, for its upcoming use in creating an extension of an already implanted spinal column support.

14. An assortment, having at least two, in particular at least four connecting devices as claimed in any one of claims 1 to 10, wherein the first fastening arrangements of at least two connecting devices have a different embodiment from one another in order to be fastened to connecting rods with different transverse dimensions, in order to be able to be placed on coupling devices of different sizes, and/or at least two connecting devices have second rods (40') of different lengths.

## Revendications

1. Dispositif de raccordement (10 ; 10') servant à raccorder une région terminale d'un premier support rachidien déjà implanté, dans lequel une pluralité de vis pédiculaires (70) sont couplées par leur côté tête à une première tige de raccordement (60) par le biais d'un dispositif de couplage (80) respectif, à une portion de support rachidien qui est déjà implantée ou à implanter, qui comporte une vis pédiculaire et qui prolonge le premier support rachidien, ledit dispositif de raccordement comportant :
un premier agencement de fixation (1) permettant une fixation du dispositif de raccordement à la première tige de raccordement (60), et
un accouplement pour la portion de support de prolongement, notamment sous la forme d'une deuxième tige (40') à coupler à la vis pédiculaire de la portion de support de prolongement, ou d'un deuxième agencement de fixation (2) permettant une fixation du dispositif de raccordement (10) à une telle deuxième tige (40) ; et comportant en outre :
un pontage rigide d'une zone intermédiaire (5) située entre le premier agencement de fixation et l'accouplement, lequel pontage épouse latéralement le dispositif de couplage (80) de la vis pédiculaire (70) du premier support rachidien déjà implanté qui est la plus proche sur le côté raccordement; ledit premier agencement de fixation (1), pour positionner le dispositif de raccordement par rapport au premier support rachidien, pouvant être mis en place radialement sur la région terminale comprenant le dispositif de couplage (80) de la vis pédiculaire la plus proche sur le côté raccordement, grâce à une faculté d'enclipsage fournie par un dispositif élastique du premier agencement de fixation, et le dispositif de raccordement comportant des moyens de serrage pour serrer la première tige de raccordement (60) contre le premier agencement de fixation (1),
**caractérisé en ce que** le dispositif élastique est conçu de telle façon qu'une délimitation latérale d'une zone de réception qui reçoit la première tige de raccordement (60) et qui présente la forme d'une rainure de réception (16) est formée par la surface intérieure d'un bras élastique (1a), et l'autre délimitation latérale, située du côté opposé, est formée par une structure latérale du premier agencement de fixation qui assure la fonction d'un bras fixe,
moyennant quoi, lors du vissage d'une vis de serrage qui est vissable dans un alésage (11) fileté ne s'étendant pas jusqu'à déboucher dans la rainure de réception (16) et qui permet de serrer la tige de raccordement (60) grâce à son agencement excentré par rapport à la rainure de réception, une force orientée dans la direction axiale est reçue de manière non centrée eu égard au centre de la tige de raccordement, vu en coupe transversale.

2. Dispositif de raccordement selon la revendication 1, dans lequel la faculté du pontage à épouser le dispositif de couplage est bilatérale.

3. Dispositif de raccordement selon la revendication 1 ou 2, dans lequel l'étendue en hauteur du pontage ne dépasse pas la hauteur du dispositif de couplage de plus de 100 %, de préférence pas de plus de 60 %, notamment pas de plus de 30 %.

4. Dispositif de raccordement selon l'une des revendications précédentes, comportant un troisième agencement de fixation (3) permettant de le fixer à la première tige de raccordement (60) à son extrémité libre côté raccordement.

5. Dispositif de raccordement selon l'une des revendications précédentes, dans lequel la possibilité de mise en place sur la région terminale est assurée par le haut.

6. Dispositif de raccordement selon la revendication 4 ou 5, dans lequel la possibilité de mise en place comprend une faculté d'enclipsage y compris du troisième agencement de fixation (3) au moyen d'un dispositif élastique (3a).

7. Dispositif de raccordement selon l'une des revendications précédentes, comportant une vis de serrage supplémentaire qui est disposée excentrée par rapport à une zone de réception (16) du troisième agencement de fixation qui reçoit la première tige de raccordement (60).

8. Dispositif de raccordement selon l'une des revendications précédentes, dans lequel le deuxième agencement de fixation (2) est conçu pour une insertion axiale, dans sa direction longitudinale, de la deuxième tige (40) et est notamment constitué sous la forme d'un alésage axial dans un bloc de matière.

9. Dispositif de raccordement selon l'une des revendications précédentes, lequel est conçu pour permettre une position sensiblement colinéaire de la première tige de raccordement (60) et de la deuxième tige (40 ; 40').

10. Dispositif de raccordement selon l'une des revendications précédentes, dont la dimension transversale maximale est inférieure à 1/3, de préférence inférieure à 2/7 de sa dimension axiale, calculée en l'absence de deuxième tige (40') éventuelle, et/ou inférieure à 440 %, notamment 360 % du diamètre de la zone de réception (16) destinée à la première tige de raccordement.

11. Ensemble de prolongement pour prolonger un premier support rachidien déjà implanté, comportant un dispositif de raccordement (10 ; 10') selon l'une des revendications précédentes et au moins une vis pédiculaire, une deuxième tige (40 ; 40'), celle-ci faisant éventuellement déjà partie du dispositif de raccordement, et au moins un dispositif de couplage pour réaliser un raccordement rigide entre la vis pédiculaire et la deuxième tige (40 ; 40').

12. Support rachidien, présentant un premier support rachidien doté d'une pluralité de vis pédiculaires couplées par leur côté tête à une première tige de raccordement (60) par le biais d'un dispositif de couplage (80) respectif, **caractérisé par** un prolongement raccordé au moyen d'un dispositif de raccordement (10 ; 10') selon une l'une des revendications 1 à 10.

13. Préparation d'un ensemble de prolongement selon la revendication 11, réalisé dans un matériau biocompatible, en vue de son utilisation prochaine pour créer un prolongement d'un support rachidien déjà implanté.

14. Assortiment comprenant au moins deux et notamment au moins quatre dispositifs de raccordement selon l'une des revendications 1 à 10, dans lequel les premiers agencements de fixation d'au moins deux dispositifs de raccordement sont de conceptions différentes l'un de l'autre, permettant leur fixation sur des tiges de raccordement de dimensions transversales différentes, leur faculté de mise en place sur des dispositifs de couplage de dimensions spatiales différentes, et/ou dans lequel au moins deux dispositifs de raccordement présentent des deuxièmes tiges (40') de longueurs différentes.
